# EUROPÄISCHE PATENTSCHRIFT

(11) **EP 2 069 515 B1**
(45) Veröffentlichungstag und Bekanntmachung des Hinweises auf die Patenterteilung: **04.04.2012**
(21) Anmeldenummer: 07815126.3
(22) Anmeldetag: 01.10.2007
(51) Int. Cl.: C12P 7/18

(54) **VERFAHREN ZUR HERSTELLUNG VON ERYTHRIT UNTER VERWENDUNG VON MONILIELLA TOMENTOSA STÄMMEN IN GEGENWART ANORGANISCHER NITRATE ALS STICKSTOFFQUELLE**
PROCESS FOR PRODUCING ERYTHRITOL USING MONILIELLA TOMENTOSA STRAINS IN THE PRESENCE OF INORGANIC NITRATES AS NITROGEN SOURCE
PROCÉDÉ DE PRODUCTION D'ÉRYTHRITOL EN UTILISANT DES SOUCHES DE MONILIELLA TOMENTOSA EN PRÉSENCE DE NITRATES INORGANIQUES COMME SOURCE D'AZOTE

(30) Priorität: 03.10.2006 AT 16462006
(43) Veröffentlichungstag der Anmeldung: 17.06.2009
(73) Patentinhaber: JUNGBUNZLAUER AUSTRIA AG, 1010 Wien (AT)
(72) Erfinder: EDLAUER, Robert, A-2136 Laa a.d. Thaya (AT); TRIMMEL, Stefan, A-2136 Laa a.d. Thaya (AT)
(74) Vertreter: Ellmeyer, Wolfgang
(86) Internationale Anmeldenummer: PCT/AT2007/000457
(87) Internationale Veröffentlichungsnummer: WO 2008/040036

(56) Entgegenhaltungen:
- EP-A- 0 136 803
- EP-A- 0 845 538

## Beschreibung

Die Erfindung betrifft ein verbessertes Verfahren zur Herstellung von Erythrit durch Gewinnung aus dem Kulturmedium einer Mikroorganismenzucht.

### Stand der Technik

Nach dem Stand der Technik sind zahlreiche Verfahren zur Produktion von Erythrit durch Mikroorganismen, vor allem durch Hefepilze bekannt, wobei verschiedenste Stickstoffquellen zum Einsatz kommen. So beschreibt etwa die JP-A 47-41549 die Verwendung eines Caseinhydrolysats als Stickstoffquelle für Stämme der Spezies *Trigonopsis* und *Candida*. JP-A 51-21072 offenbart die Verwendung von Hefeextrakt und Harnstoff für verschiedene Hefestämme.

JP-A 60-110295 und JP-A 1-199584 beschreiben jeweils die Verwendung von *Moniliella tomentosa* var. *pollinis* zur Produktion von Erythrit. Da bekannt ist, dass diese Varietät hohe Nährstoffansprüche aufweist, enthalten die dafür bekannten Kulturmedien üblicherweise einen hohen Anteil an komplexen Stickstoffquellen wie Hefeextrakt, Pepton, Maisquellwasser oder ähnlichen proteinreichen Substanzen pflanzlicher oder tierischer Herkunft. In JP-A 60-110295 und JP-A 1-199584 werden daher Maisquellwasser, Harnstoff und Hefeextrakt als Stickstoffquellen eingesetzt,

Diese komplexen Stickstoffquellen stellen jedoch nicht nur einen entscheidenden Kostenfaktor dar, sondern erschweren auch die Reinigung des gebildeten Erythrits und verringern die Ausbeute bei der Kristallisation. Es wurden daher umfangreiche Bemühungen unternommen, den Anteil an komplexen Stickstoffquellen so weit wie möglich zu reduzieren. Dies kann durch Verwendung organischer Stickstoffquellen wie Harnstoff oder auch von anorganischen Salzen wie Ammoniumsulfat geschehen. Da bei der Fermentation von Erythrit nicht unerhebliche Mengen Ethanol gebildet werden, ist die Verwendung von Harnstoff durch die mögliche Bildung des als kanzerogen eingestuften Ethylcarbamats problematisch (G. Haßelbeck, W. Henke; Erbslöh-Weinkompendium Enzyme; Erbslöh Geisenheim Getränketechnologie; D-65366 Die sich mit dieser Problematik befassende EP 0.845.538 B1 sowie deren deutsche Übersetzung DE 697 20 379 T2 offenbaren daher die Verwendung von Ammoniumsulfat als anorganisches Salz zusammen mit Maisquellwasser, wobei Ersteres als "Hauptstickstoffquelle" bezeichnet wird und 50 bis 85 % der Stickstoffquellen ausmacht, während Letzteres die übrigen 15 bis 50 % darstellt. Als Hefestämme werden neben *Moniliella* (auch "*Moniliella pollinis*") auch Vertreter von *Yarrowia* und *Trichosporonoides* genannt. Als ein Verfahrensschritt wird die Einstellung des pH-Werts auf Werte zwischen 3 und 7 genannt (siehe u.a. den dortigen Anspruch 12). Die potenzielle Verwendbarkeit von Kaliumnitrat als Stickstoffquelle wird allgemein in einer Tabelle erwähnt.

Aus der EP-A-0.136.802 war freilich seit 1985 bekannt, dass die Polyolbildung durch *Moniliella tomentosa* var. *pollinis* in einem pH-Bereich von 3 bis 6, vorzugsweise in einem pH-Bereich von 4 bis 5, in wünschenswerter Weise abläuft. Die Einflüsse des pH-Werts auf die Polyolausbeute wurden bereits 1972 von L. Hanssens, A. van Regenmortel und H. Verachtern beschrieben (Applied Microbiology, Nov. 1972, S. 831-833).

Die in der EP 0.845.538 B1 beschriebene Verwendung von Ammoniumsulfat, das ein Salz einer sehr starken zweibasigen Säure (pK_{A} = -3 und 1,9) und einer vergleichsweise schwachen Base (pK_{B} = 4,75, d.h. pK_{A} = 9,25) ist, erfordert somit zwingend die Regelung des pH-Werts, da sonst der pH durch die Freisetzung von Schwefelsäure auf einen für das Wachstum und die Produktion ungünstigen Wert von unter 3, mitunter sogar unter 2, abfällt. EP 0.845.538 B1 löst das durch automatische Zudosierung von 35%iger Natronlauge, was einen erheblichen regelungstechnischen Mehraufwand darstellt und bei einem Versagen zu einem Totalausfall der Fermentation führen kann.

Ein weiterer Aspekt bei der Fermentation zur Erythritgewinnung ist die Unterdrückung von Nebenprodukten wie Ribit und Glycerin. In EP 0.845.538 wird hierzu beschrieben, dass ein höherer Anteil an komplexen Stickstoffquellen (in diesem Fall Maisquellwasser) die Bildung von Glycerin anregt. Eine Limitierung der komplexen Wuchsstoffquellen geht aber immer mit einer Verminderung der Wachstums- und Produktionsgeschwindigkeit einher. Das Verfahren gemäß EP 0.845.538, worin neben Ammoniumsulfat bis zu 50 % Maisquellwasser als Stickstoffquelle eingesetzt werden, löst dieses Problem der Nebenproduktbildung jedoch unzureichend, wie die in den dortigen Ausführungsbeispielen nicht vollständig unterdrückbare Bildung von Glycerin zeigt: Die gebildete Menge an Glycerin, bezogen auf Erythrit, schwankt zwischen etwa 1,9 % (Beispiel 5) und 50,2 % (Beispiel 12).

In Biotechnology and Bioengineering vom 5. August 2006, S. 899-908, untersuchten Diano et al. den Einfluss verschiedener Parameter, u.a. jenen der Stickstoffquellen Ammonium und Nitrat, auf den Stoffwechsel von *Aspergillus niger*. Der pH-Wert wurde dabei anfänglich auf 3 eingestellt und anschließend schrittweise auf 4,5 erhöht. Als Ergebnis wird festgestellt, dass sich Nitrat gegenüber Ammonium positiv auf die Produktion mancher Alkohole, u.a. von Erythrit, durch *Aspergillus* auswirkt.

In der US-A-6.030.820 wird ein Verfahren zur Herstellung von hochreinem kristallinem Erythrit aus einer Mikrobenkulturlösung als Ausgangsmaterial beschrieben. Als ein möglicher Mikroorganismus zum Erhalt des Ausgangsmaterials werden *Moniliella tomentosa var. pollinis* und als mögliche Stickstoffquelle Ammoniumnitrat beschrieben. Weder darauf noch auf den pH-Wert während der Kultivierung wird näher eingegangen.

Auch die EP 0.327.342 A2 offenbart ein Verfahren zur Herstellung von Erythrit durch Kultivieren eines diesen produzierenden Mikroorganismus, wobei die Zellkonzentration im Fermenter mittels Zyklieren eines Teils der Kulturbrühe durch einen äußeren Zellseparator kontinuierlich gesteuert wird. Ammoniumnitrat ist ganz allgemein als mögliche Stickstoffquelle aufgelistet. Als einziges Beispiel für den Mikroorganismus wird *Aureobasidium sp. SN-G42* genannt, und lediglich im letzten Ausführungsbeispiel wird ein pH-Wert des Kulturmediums angegeben (pH 4,2), ansonsten jedoch nicht erwähnt.

In Biotechnology and Bioprocess Engineering 8(3), 173-178 (2003), beschreiben Lee, Kwang-Jun et al. die Verbesserung der Erythrit-Produktivität bei Kultivierung verschiedener *Penicillium*-Mutanten. Für eine davon wird erwähnt, dass durch Zusatz von Ammoniumcarbonat, Kaliumnitrat und Natriumnitrat die Ausbeute an Erythrit beträchtlich erhöht werden konnte. Als Beispiel wird in der CAS-Zusammenfassung ein Versuch mit 0,5 % Hefeextrakt, 0,5 % (NH₄)₂C₂O₄ (offenbar Ammoniumoxalat), 0,1 % KNO₃ und 0,1 % NaNO₃ als Stickstoffquellen angeführt (wobei unter den Stickstoffquellen somit die beiden Nitrate einen Gewichtsanteil von unter 17 % und einen Anteil an verwertbarem Stickstoff von etwa 15 % ausmachen).

### Aufgabe der Erfindung

Aufgabe der Erfindung war es daher, ein verbessertes, kostengünstiges Verfahren zur Produktion von Erythrit in hoher Ausbeute und Reinheit bereitzustellen, das die obigen Probleme hinsichtlich des pH-Werts des Kulturmediums und der Bildung von Nebenprodukten zumindest abschwächt oder sogar gänzlich beseitigt.

### Offenbarung der Erfindung

Dieses Ziel wird durch die erfindungsgemäße Verwendung zumindest eines anorganischen Nitrats in einem Fermentationsverfahren zur Herstellung von Erythrit unter Einsatz eines Hefestamms der Spezies *Moniliella* tomentosa als Erythrit produzierender Mikroorganismus, wobei das zumindest eine anorganische Nitrat gleichzeitig sowohl als Hauptstickstoffquelle als auch als pH-Regler im Kulturmedium dient, sowie durch ein solcherart verbessertes Verfahren zur Herstellung von Erythrit erreicht. In einem erfindungsgemäßen Verfahren wird in bekannter Weise ein Hefestamm der Spezies *Moniliella* tomentosa in einem eine oder mehrere Kohlenstoff- und eine oder mehrere Stickstoffquellen enthaltenden Kulturmedium gezüchtet, wobei zumindest ein anorganisches Nitrat als Stickstoffquelle im Kulturmedium eingesetzt wird, und Erythrit aus dem Medium gewonnen. Das erfindungsgemäße Verfahren weist analog zur erfindungsgemäßen Verwendung das Kennzeichen auf, dass das zumindest eine anorganische Nitrat gleichzeitig sowohl als Hauptstickstoffquelle als auch als pH-Regler im Kulturmedium eingesetzt wird. Das zumindest eine anorganische Nitrat wird vorzugsweise aus Kaliumnitrat, Natriumnitrat und Ammoniumnitrat, insbesondere Kaliumnitrat und Natriumnitrat, ausgewählt.

Bei Verwendung dieses zumindest einen anorganischen Nitrats als Hauptstickstoffquelle können somit einerseits Verfahrensschritte zur pH-Einstellung entfallen, da der pH-Wert durch die geeignete Wahl der Art und Menge an Nitrat geregelt wird und während der Fermentation auch ohne Nachregelung stets im optimalen Bereich bleibt. Andererseits wird die Bildung von anderen Alkoholen wie Glycerin als Nebenprodukte drastisch unterdrückt und kann in bevorzugten Ausführungsformen sogar gänzlich ausgeschaltet werden. Die gemäß vorliegender Erfindung eingesetzten anorganischen Nitrate sind - im Gegensatz zu Harnstoff - zugelassene Lebensmittelzusatzstoffe (E251, E252), die zudem im Verlauf der Fermentation innerhalb der ersten 2 bis 3 Tage vollständig abgebaut werden.

Als "Hauptstickstoffquelle" ist hierin eine solche zu verstehen, die zumindest 20 % des verwertbaren Stickstoffs bereitstellt. Die Vorteile der Erfindung treten jedoch vor allem bei Einsatz des zumindest einen anorganische Nitrats in einer Menge von 30 bis 85 %, noch bevorzugter 45 bis 65 %, des verwertbaren Gesamtstickstoffgehalts zu Tage, wobei die jeweils optimale Menge auch von der Basizität des Kations abhängt.

Erfindungsgemäß können zusätzlich zu dem zumindest einen anorganischen Nitrat auch eine oder mehrere organische Stickstoffquellen, z.B. Maisquellwasser, Hefeextrakt und Gemische davon, sogar in relativ hohen Anteilen eingesetzt werden, ohne dass die Bildung von Nebenprodukten in unerwünschtem Ausmaß auftritt. Entgegen der Lehre wurde vielmehr festgestellt, dass mit steigenden Konzentrationen an Maisquellwasser der Anteil an Nebenprodukten sogar sinkt. Bei erfindungsgemäßer Verwendung anorganischer Nitrate kann der Anteil der komplexen Stickstoffquelle sowohl an die Bedürfnisse der Fermentation, d.h. möglichst hohe Produktivität und Ausbeute, als auch an die der Produktisolierung, d.h. möglichst wenig störende Bestandteile und Nebenprodukte, optimal angepasst werden.

Als Mikroorganismen werden erfindungsgemäß Vertreter der Spezies *Moniliella tomentosa*, bevorzugt ein Stamm der Varietät *pollinis,* eingesetzt, um aufgrund der bei diesen Pilzen besonders gut zur Geltung kommenden Wirkung der Erfindung Erythrit in möglichst hoher Ausbeute und Reinheit zu erhalten.

Die vorliegende Erfindung wird nachstehend unter Bezugnahme auf konkrete Ausführungsbeispiele beschrieben, die zur Illustration dienen.

### Beispiele

### Beispiel 1

100 ml eines Vorkulturmediums, bestehend aus

| | |
|---|---|
| 15 % | Glucose |
| 0,4 % | KNO₃ |
| 0,1 | % Hefeextrakt |
| 0,6 % | Maisquellwasser |
| 10 ppm | MnSO₄.H₂O |
| 2 ppm | Thiamin-hydrochlorid |

wurde mit Zitronensäure auf pH 3,6 eingestellt und in einem 500-ml-Rundkolben 20 min lang bei 110 °C sterilisiert. Der auf 30 °C abgekühlte Kolben wurde aus einer Glycerinkonserve mit *Moniliella tomentosa* var. *pollinis* beimpft und 24 Stunden bei 30 °C und 200 min⁻¹ geschüttelt.

1 ml dieser Vorkultur wurde steril auf 100 ml Produktionsmedium, bestehend aus

| | |
|---|---|
| 35 % | Glucose |
| 0,35 % | KNO₃ |
| 0,05 % | Hefeextrakt |
| 0,40 % | Maisquellwasser |
| 0,025 % | KH₂PO₄ |
| 5 ppm | MnSO₄.H₂O |
| 3 ppm | Thiamin-hydrochlorid |

überimpft. Das Medium wurde in vier 500-ml-Schüttelkolben vor dem Beimpfen mit Zitronensäure auf pH 3,6 eingestellt, 20 min lang bei 110 °C sterilisiert und auf 30 °C temperiert. Die Kolben wurden 9 Tage lang mit 200 min⁻¹ geschüttelt. Während der Dauer der Fermentation wurden steril Proben entnommen und mittels HPLC analysiert. In diesem Beispiel wurden 62 % des verwertbaren Gesamtstickstoffgehalts des Mediums in Form von Kaliumnitrat zugesetzt.

Nachstehend sind die HPLC-Analysenergebnisse nach jeweils 0, 42, 116, 165 und 210 Stunden Fermentation für die vier Testkolben angeführt.

| Kolben 1 | | | | | Kolben 2 | | | |
|---|---|---|---|---|---|---|---|---|
| Dauer (h) | % Glucose | % Erythrit | pH | | Dauer (h) | % Glucose | % Erythrit | pH |
| 0 | 35,00 | 0,00 | 3,60 | | 0 | 35,00 | 0,00 | 3,60 |
| 42 | 27,86 | 0,36 | 5,24 | | 42 | 27,99 | 0,35 | 5,24 |
| 116 | 12,11 | 7,06 | 4,25 | | 116 | 12,92 | 7,44 | 4,26 |
| 165 | 4,23 | 12,80 | 3,95 | | 165 | 4,54 | 12,87 | 3,96 |
| 210 | 0,00 | 13,71 | 4,00 | | 210 | 0,00 | 14,52 | 4,01 |

| Kolben 3 | | | | | Kolben 4 | | | |
|---|---|---|---|---|---|---|---|---|
| Dauer (h) | % Glucose | % Erythrit | pH | | Dauer (h) | % Glucose | % Erythrit | pH |
| 0 | 35,00 | 0,00 | 3,60 | | 0 | 35,00 | 0,00 | 3,60 |
| 42 | 27,86 | 0,37 | 5,22 | | 42 | 27,32 | 0,36 | 5,24 |
| 116 | 12,90 | 7,59 | 4,27 | | 116 | 12,75 | 7,66 | 4,26 |
| 165 | 4,29 | 13,04 | 3,97 | | 165 | 3,96 | 12,66 | 3,97 |
| 210 | 0,00 | 15,25 | 4,04 | | 210 | 0,00 | 14,26 | 4,04 |

Der Mittelwert des Erythritgehalts in den vier Kolben betrug 14,4 %. Der pH-Wert verblieb während der gesamten Fermentationsdauer ohne jeglichen Basenzusatz im optimalen Bereich von 3 bis 6.

### Vergleichsbeispiel 1

Der Versuch aus Beispiel 1 wurde wiederholt, wobei das Produktionsmedium jedoch 0,23 % (NH₄)₂SO₄ anstelle von 0,35 % KNO₃ enthielt. Der Anteil an anorganischem Stickstoff wurde in beiden Versuchen mit etwa 0,05 % Stickstoff gleich gehalten und entsprach 62 % der verwertbaren Gesamtstickstoffmenge.

| Kolben 1 | | | | | Kolben 2 | | | |
|---|---|---|---|---|---|---|---|---|
| Dauer (h) | % Glucose | % Erythrit | pH | | Dauer (h) | % Glucose | % Erythrit | pH |
| 0 | 35,00 | 0,00 | 3,60 | | 0 | 35,00 | 0,00 | 3,60 |
| 42 | 28,75 | 0,31 | 2,15 | | 42 | 30,62 | 0,29 | 2,11 |
| 116 | 26,19 | 1,13 | 2,00 | | 116 | 25,13 | 0,90 | 1,96 |
| 165 | 25,18 | 1,26 | 2,01 | | 165 | 25,18 | 0,93 | 1,97 |
| 210 | 26,77 | 1,47 | 1,99 | | 210 | 22,82 | 0,85 | 1,95 |

| Kolben 3 | | | | | Kolben 4 | | | |
|---|---|---|---|---|---|---|---|---|
| Dauer (h) | % Glucose | % Erythrits | pH | | Dauer (h) | % Glucose | % Erythrit | pH |
| 0 | 35,00 | 0,00 | 3,60 | | 0 | 35,00 | 0,00 | 3,60 |
| 42 | 26,41 | 0,28 | 2,12 | | 42 | 30,93 | 0,31 | 2,12 |
| 116 | 25,69 | 0,98 | 1,96 | | 116 | 24,52 | 0,95 | 1,94 |
| 165 | 28,37 | 1,07 | 1,98 | | 165 | 26,83 | 1,06 | 1,98 |
| 210 | 21,50 | 0,84 | 1,96 | | 210 | 26,90 | 1,06 | 1,93 |

Wie aus den obigen Werten hervorgeht, fällt durch die Assimillation von Ammonium der pH-Wert in der Wachstumsphase auf Werte von etwa 2 und darunter ab. Eine Produktion ohne pH-Regelung ist also nicht möglich. Die Mengen an gebildetem Erythrit sind dementsprechend niedrig und betragen bestenfalls nur etwa 1/10 jener im erfindungsgemäßen Beispiel 1.

### Beispiel 2

Beispiel 1 wurde wiederholt, wobei die Mengen an Maisquellwasser (MQW) (0,40 %) und an Kaliumnitrat (0,35 %) aus Beispiel 1 jeweils sowohl gleich belassen, verdoppelt als auch halbiert wurden. Nach 10 Tagen wurden die Konzentrationen von Glycerin und Ribit sowie die Ausbeute an Erythrit, bezogen auf die eingesetzte Glucose, bestimmt. Jeder Versuch wurde in Doppelansätzen durchgeführt, deren Mittelwerte nachstehend angegeben sind.

| **Glycerin (%)** | | | | | **Ribit (%)** | | | |
|---|---|---|---|---|---|---|---|---|
| **KNO₃/ MQW** | **0,20%** | **0,40 %** | **0,80 %** | | **KNO₃ / MQW** | **0,20 %** | **0,40 %** | **0,80 %** |
| **0,18 %** | 6,63 | 4,68 | 0,00 | | **0,18 %** | 3,66 | 3,88 | 0,80 |
| **0,35 %** | 3,31 | 0,70 | 0,00 | | **0,35 %** | 2,32 | 2,26 | 0,36 |
| **0,70%** | 0,78 | 0,00 | 0,00 | | **0,70%** | 0,16 | 0,15 | 0,16 |

| **Ausbeute an Erythrit (%)** | | | | | **Produktivität (g/l.h)** | | | |
|---|---|---|---|---|---|---|---|---|
| **KNO₃ / MQW** | **0,20 %** | **0,40 %** | **0,80 %** | | **KNO₃ / MQW** | **0,20 %** | **0,40 %** | **0,80 %** |
| **0,18%** | 26,0 | 38,1 | 49,8 | | **0,18 %** | 0,55 | 0,96 | 1,21 |
| **0,35 %** | 42,5 | 50,0 | 50,1 | | **0,35%** | 0,88 | 1,06 | 1,38 |
| **0,70 %** | 42,3 | 45,7 | 44,0 | | **0,70 %** | 0,96 | 1,27 | 1,31 |

Es ist offensichtlich, dass unter Verwendung von anorganischem Nitrat als Hauptstickstoffquelle mit zunehmenden Mengen an Maisquellwasser gleichzeitig sowohl die Nebenproduktbildung unterdrückt als auch die Ausbeute gesteigert werden kann. Durch das erfindungsgemäße Verfahren kann Glycerin als Nebenprodukt gänzlich vermieden werden, während die Menge an gebildetem Ribit weit unter 1 % gedrückt werden kann. Zusätzlich steigt Produktivität mit höheren Stickstoffgaben merklich an.

### Beispiel 3

50 ml einer Vorkultur wie in Beispiel 1 beschrieben wurden auf einen 3-Liter-Fermenter mit einem Medium, bestehend aus

| | |
|---|---|
| 34 % | Glucose |
| 0,20 % | KNO₃ |
| 0,08 % | NH₄NO₃ |
| 0,05 % | Hefeextrakt |
| 0,30 % | Maisquellwasser |
| 0,025 % | KH₂PO₄ |
| 3 ppm | MnSO₄.H₂O |
| 3 ppm | Thiamin-hydrochlorid |

überimpft. Die Fermentation erfolgte bei einer Temperatur von 28 °C ohne pH-Regelung. Die Belüftung betrug 0,18 vvm bei einer Rührerdrehzahl von 500 U.min⁻¹. Nachdem die Glucose verbraucht war, wurden folgende Werte gefunden:

| | |
|---|---|
| Erythrit | 16,11 % |
| Ribit | 0,26 % |
| Glycerin | 0,00 % |
| pH-Wert | 3,57 |

Somit wurde bewiesen, dass ein Teil des Kaliumnitrats auch durch andere anorganische Nitrate ersetzt werden kann, ohne die oben beschriebenen und durch Messwerte belegten Vorteile der Erfindung zu beeinträchtigen.

### Beispiel 4

80 Liter einer Vorkultur wie in Beispiel 1 beschrieben wurden auf eine 3.000-Liter-Blasensäule mit 2.600 Litern eines Mediums, bestehend aus

| | |
|---|---|
| 23,01 % | Glucose |
| 17,25 % | Fructose |
| 0,35 % | KNO₃ |
| 0,05 % | Hefeextrakt |
| 0,40 % | Maisquellwasser |
| 0,025 % | KH₂PO₄ |
| 5 ppm | MnSO₄.H₂O |
| 3 ppm | Thiamin-hydrochlorid |

überimpft. Die Temperatur wurde auf 30 °C geregelt. Die Belüftung wurde auf 0,19 vvm eingestellt. Nachdem der Zucker vollständig verbraucht war, wurden folgende Werte gefunden:

| | |
|---|---|
| Erythrit | 19,96 % |
| Ribit | 0,18 % |
| Glycerin | 1,96 % |
| pH-Wert (Mittelwert) | 4,48 |
| Ausbeute an Erythrit | 49,4 % |

Der pH-Wert schwankte zwischen 4,35 und 4,62 und lag somit ohne jegliche Regelung im optimalen Bereich. Während der Fermentation wurde zu keinem Zeitpunkt eine Zugabe von Antischaummittel notwendig. Aus diesem Beispiel geht somit hervor, dass auch andere Kohlenstoffquellen als Glucose im erfindungsgemäßen Verfahren unter Verwendung von anorganischem Nitrat als Hauptstickstoffquelle mit der gleichen Effizienz verwertet werden können.

Zusammenfassend stellt die vorliegende Erfindung durch die Verwendung zumindest eines anorganischen Nitrats sowohl als Hauptstickstoffquelle als auch als pH-Regler somit Verfahren zur Produktion von Erythrit bereit, durch die das gewünschte Produkt in hoher Ausbeute und Reinheit sowie auf kostengünstige Weise erhalten werden kann: a) Verfahrensschritte zur pH-Wert-Regelung können gänzlich entfallen; b) es werden äußerst geringe Mengen an störenden Nebenprodukten gebildet, was die Isolierung des Erythrits deutlich vereinfacht; c) die Produktivität kann erheblich gesteigert werden; und d) die eingesetzten anorganischen Nitrate sind zu günstigen Preisen im Handel erhältlich. Daher besteht auch keinerlei Zweifel an der gewerblichen Anwendbarkeit der Erfindung.

## Patentansprüche

1. Verwendung zumindest eines anorganischen Nitrats in einem Fermentationsverfahren zur Herstellung von Erythrit unter Einsatz eines Hefestamms der Spezies *Monilialla tomentosa* als Erythrit produzierender Mikroorganismus,
**dadurch gekennzeichnet, dass** das zumindest eine anorganische Nitrat gleichzeitig sowohl als Stickstoffquelle, die zumindest 20 % des verwertbaren. Gesamtstickstoffgehalts bereitstellt, als auch als pH-Regler im Kulturmedium dient.

2. Verfahren zur Herstellung von Erythrit, umfassend das Züchten eines Hefestamms der Spezies *Moniliella tomentosa* in einem eine oder mehrere Kohlenstoff- und eine oder mehrere Stickstoffquellen enthaltenden. Kulturmedium, wobei zumindest ein anorganisches Nitrat als Stickstoffquelle im Kulturmedium eingesetzt wird, sowie das Gewinnen des Erythrits aus dem Medium,
**dadurch gekennzeichnet, dass** das zumindest eine anorganische Nitrat gleichzeitig sowohl als Stickstoffquelle, die zumindest 20 % des verwertbaren Gesamtstickstoffgehalts bereitstellt, als auch als pH-Regler im Kulturmedium eingesetzt wird.

3. Verwendung nach Anspruch 1 bzw. Verfahren nach Anspruch 2, **dadurch gekennzeichnet, dass** das zumindest eine anorganische Nitrat aus Kaliumnitrat, Natriumnitrat und Ammoniumnitrat ausgewählt wird.

4. Verwendung bzw. Verfahren nach Anspruch 3, **dadurch gekennzeichnet, dass** das zumindest eine anorganische Nitrat aus Kaliumnitrat und Natriumnitrat ausgewählt wird.

5. Verwendung bzw. Verfahren nach einem der vorangegangenen Ansprüche, **dadurch gekennzeichnet, dass** das zumindest eine anorganischen Nitrat in einer Menge von 30 bis 85 % des verwertbaren Gesamtstickstoffgehalts eingesetzt wird.

6. Verwendung bzw. Verfahren nach Anspruch 5, **dadurch gekennzeichnet, dass** das zumindest eine anorganischen Nitrat in einer Menge von 45 bis 65 % des verwertbaren Gesamtstickstoffgehalts eingesetzt wird.

7. Verwendung bzw. Verfahren nach einem der vorangegangenen Ansprüche, **dadurch gekennzeichnet, dass** zusätzlich zu dem zumindest einen anorganischen Nitrat eine organische Stickstoffverbindung als weitere Stickstoffquelle eingesetzt wird.

8. Verwendung bzw. Verfahren nach Anspruch 7, **dadurch gekennzeichnet, dass** Maisquellwasser und/oder Hefeextrakt als weitere Stickstoffquelle eingesetzt wird.

9. Verwendung bzw. Verfahren nach einem der vorangegangenen Ansprüche, **dadurch gekennzeichnet, dass** ein Stamm von *Moniliella tomentosa* der Varietät *pollinis* eingesetzt wird.

## Claims

1. A use of at least one inorganic nitrate in a fermentation process for producing erythritol using a yeast strain of the *Moniliella tomentosa* species as an erythritol-producing microorganism,
**characterized in that** the at least one inorganic nitrate is simultaneously used as a nitrogen source providing at least 20 % of the total utilizable nitrogen content and as a pH regulator in the culture medium.

2. A method for producing erythritol, which comprises growing a yeast strain of the *Moniliella tomentosa* species in a culture medium containing one or more carbon source(s) and one ore more nitrogen source(s), wherein at least one inorganic nitrate is used as a nitrogen source in the culture medium, as well as recovering erythritol from the medium,
**characterized in that** the at least one inorganic nitrate is simultaneously used as a nitrogen source providing at least 20 % of the total utilizable nitrogen content and as a pH regulator in the culture medium.

3. The use according to claim 1 or the method according to claim 2, **characterized in that** the at least one inorganic nitrate is selected from potassium nitrate, sodium nitrate and ammonium nitrate.

4. The use or method according to claim 3, **characterized in that** the at least one inorganic nitrate is selected from potassium nitrate and sodium nitrate.

5. The use or method according to any one of the preceding claims, **characterized in that** the at least one inorganic nitrate is used in an amount of 30 to 85 % of the total utilizable nitrogen content.

6. The use or method according to claim 5, **characterized in that** the at least one inorganic nitrate is used in an amount of 45 to 65 % of the total utilizable nitrogen content.

7. The use or method according to any one of the preceding claims, **characterized in that**, in addition to the at least one inorganic nitrate, an organic nitrogen source is used as a further nitrogen source.

8. The use or method according to claim 7, **characterized in that** corn steep water and/or yeast extract are/is used as (a) further nitrogen source(s).

9. The use or method according to any one of the preceding claims, **characterized in that** a strain of *Moniliella tomentosa* of the *pollinis* variety is used.

## Revendications

1. Utilisation d'au moins un nitrate inorganique dans un procédé de fermentation pour la production d'érythrite en utilisant une souche de levure de l'espèce *Moniliella tomentosa* comme microorganisme qui produit d'érythrite,
**caractérisée en ce que** ledit au moins un nitrate inorganique sert de source d'azote, fournissant au moins 20 % de la teneur exploitable totale en azote, et en même temps de régulateur de pH dans le milieu de culture.

2. Procédé pour la production d'érythrite, comprenant la culture d'une souche de levure de l'espèce *Moniliella tomentosa* dans un milieu de culture contenant une ou plusieurs sources de carbone et une ou plusieurs sources d'azote, au moins un nitrate inorganique étant utilisé comme source d'azote dans le milieu de culture, ainsi que l'extraction de l'érythrite dudit milieu,
**caractérisé en ce que** ledit au moins un nitrate inorganique sert de source d'azote, fournissant au moins 20 % de la teneur exploitable totale en azote, et en même temps de régulateur de pH dans le milieu de culture.

3. Utilisation selon la revendication 1 ou 2, **caractérisée en ce que** ledit au moins un nitrate inorganique est choisi du nitrate de potassium, du nitrate de sodium et du nitrate d'ammonium.

4. Utilisation ou procédé selon la revendication 3, caractérisé/e en ce que ledit au moins un nitrate inorganique est choisi du nitrate de potassium et du nitrate de sodium.

5. Utilisation ou procédé selon l'une des revendications précédentes, caractérisé/e en ce que ledit au moins un nitrate inorganique est utilisé en une quantité de 30 à 85 % de la teneur exploitable totale en azote.

6. Utilisation ou procédé selon la revendication 5, caractérisé/e en ce que ledit au moins un nitrate inorganique est utilisé en une quantité de 45 à 65 % de la teneur exploitable totale en azote.

7. Utilisation ou procédé selon l'une des revendications précédentes, caractérisé/e en ce qu'un composé organique d'azote est utilisé en plus dudit au moins un nitrate inorganique comme source d'azote additionnelle.

8. Utilisation ou procédé selon la revendication 7, caractérisé/e en ce que du liqueur de maïs et/ou de l'extrait de levure est/sont utilisé/s comme source/s d'azote additionnelle/s.

9. Utilisation ou procédé selon l'une des revendications précédentes, caractérisé/e en ce qu'une souche de l'espèce *Moniliella tomentosa* de la variété *pollinis* est utilisée.
